# EUROPEAN PATENT SPECIFICATION

(11) **EP 0 435 946 B1**
(45) Date of publication and mention of the grant of the patent: **04.01.1995**
(21) Application number: 89911334.4
(22) Date of filing: 13.09.1989
(51) Int. Cl.: A61K 33/32, A61K 37/12, G01N 33/53, G01N 33/68, C07K 3/20, A61K 37/02

(54) **METHODS FOR MODIFYING THE BINDING OF CELL ADHESION RECEPTORS**
METHODE FÜR DIE ÄNDERUNG DER BINDUNG EINES ZELLADHÄSIONREZEPTORS
PROCEDE DE MODIFICATION DE LA LIAISON DE RECEPTEURS D'ADHESION DE CELLULES

(30) Priority: 14.09.1988 US 244701
(43) Date of publication of application: 10.07.1991
(73) Proprietor: LA JOLLA CANCER RESEARCH FOUNDATION, La Jolla, CA 92037 (US)
(72) Inventor: RUOSLAHTI, Erkki, I., Rancho Santa Fe, CA 92067 (US); GAILIT, James, O., New York 11754 (US); GEHLSEN, Kurt, R., San Diego, CA 92109 (US); HAUTANEN, Aarno, Helsinki 00640 (FI); PIERSCHBACHER, Michael, D., San Diego, CA 92107 (US)
(74) Representative: UEXKÜLL & STOLBERG
(86) International application number: US8903979
(87) International publication number: WO9002556

(56) References cited:
- US-A- 4 578 079
- US-A- 4 614 517
- US-A- 4 683 291
- Experimental Cell Research 110(1977), pp. 419-425
- Advan. in Biophys., Vol. 6, pp. 157-181 (1974)
- Clinical Research 13:237 (1965), S. Lipson et al, Multivalent Cations and Cell Adhesion
- J. Cell Sci., Vol. 65, 1984, Frederick Grinnell; see page 61 - 72
- Science, Vol. 238, 1987, Erkki Ruoslahti and Michael D. Pierschbacher; see page 491 - 497
- The Journal of Biological Chemistry, Vol. 263, No. 26, 1988, (U.S.A.) James Gailit and Erkki Ruoslahti; see pages 12927-12932
- Neuron, Vol. 1, 1988, Michael J. Ignatius and Lois F. Reichardt; see page 713 - 725

## Description

Cell adhesion plays a critical role in the maintenance and promotion of cell anchorage, polarity, migration, and differentiation. Adhesion processes are mediated by cell surface receptors which specifically recognize and bind to adhesion ligands.

Many of the known adhesion receptors belong to a family of receptors called integrins. The integrin family includes receptors for fibronectin, vitronectin, and fibrinogen as well as receptors for von Willebrand factor, laminin, complement component C3bi and some cell-cell adhesion receptors. The integrins are heterodimeric membrane proteins with large extracellular domains and small cytoplasmic tails. The ligand proteins of those integrins with known ligands share one common feature: the tripeptide Arg-Gly-Asp (RGD) is an essential part of the protein structure recognized by the receptor. Integrin-mediated adhesion processes can be manipulated by providing either immobilized or soluble Arg-Gly-Asp containing peptides. The ability to manipulate such processes has enormous potential application. The characteristics and uses of Arg-Gly-Asp containing peptides are described in, for example, U.S. Pat. Nos. 4,578,079 and 4,614,517 and in Pierschbacher and Ruoslahti, Nature 309:30 (1984) and Pierschbacher and Ruoslahti, PNAS 81:5985 (1984).

The binding of adhesion receptors to their ligands requires the presence of divalent cations such as Ca²⁺ or Mg²⁺. The alpha subunits of the receptors contain several short amino acid sequences that are similar to the Ca²⁺-binding sites of other known Ca²⁺-binding proteins and the divalent cations presumably act through these sequences.

Because of the critical role played by cell adhesion processes, there exists a need to be able to further manipulate such systems. The present invention satisfies this need and provides related advantages as well. It was already known to use zinc and aluminium ions in wound healing compositions (Rota liste, 1976, 31551B and 31512B). Zinc salts have been reported in Martindale "The Extra Pharmacopoeia", 28th Ed, p.945 to assist wound-healing.

The present invention resides in the finding that the binding avidity of cell surface receptors for their ligands can be modified by providing certain cations. Preferably, the receptor is the fibronectin, vitronectin, laminin, or a collagen receptor, although other integrins can also be used. Among the cations which result in an increase in binding avidity are the cations Mn²⁺, Co²⁺, Cd²⁺ and Ni²⁺. According to the invention the cation is not Zn²⁺ or Al³⁺, nor a physiological concentration of Ca²⁺ or Mg²⁺. Gd³⁺ results in decreased avidity of the fibronectin and vitronectin receptors for their ligands. Based on this concept the invention refers to the use of cations having integrin-binding-modifying activity for the manufacture of a medicament for modifying the binding avidity of integrins. The inventive concept can further be employed in the context with a ligand matrix column in order to increase the yield of receptors purified therewith. The invention can also be used to increase the binding of receptors to a substrate, as in an ELISA or radioreceptor assay, and to increase the migration of cells.

Figure 1 shows the effect of cations on the binding of fibronectin receptor liposomes to fibronectin. Receptor liposomes prepared without added cations were mixed with the indicated concentrations of CaCl₂, MgCl₂ or MnCl₂ and their ability to attach to plastic microtiter wells coated with fibronectin was assayed. The results from three experiments are expressed as a percentage of control attachment, which was measured in the presence of 1 mM CaCl₂ and 1 mM MgCl₂ and set equal to 100%.

Figure 2 shows attachment of fibronectin receptor liposomes to fibronectin in the presence of the 110 kDa fibronectin fragment. Receptor liposomes were prepared with either 1 mM CaCl₂ and 1 mM MgCl₂ or with 1 mM MnCl₂. The average of results from three experiments is expressed as percentage of control attachment measured in the presence of appropriate cations without inhibitor.

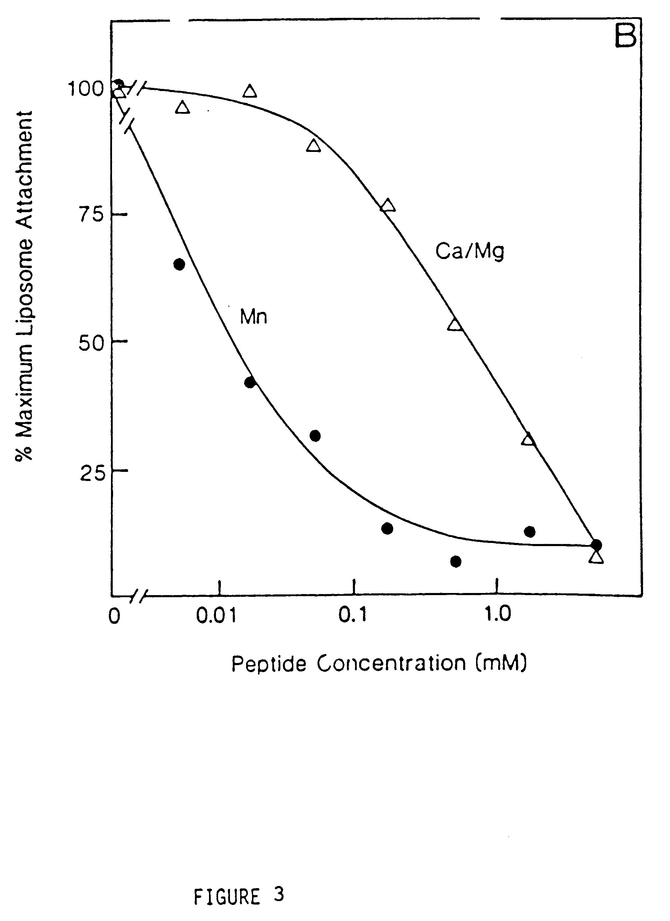

Figure 3 shows attachment as described in Figure 2 using as the soluble inhibitor GRGDSP.

Figure 4 shows the binding of the fibronectin receptor to fibronectin in ELISA and inhibition of the binding by soluble ligands. Microtiter wells were coated with 1 µg/ml of fibronectin, and purified receptor was allowed to bind to the coated wells in the presence of increasing concentrations of fibronectin, GRGDSP, GRGESP or an unrelated peptide. The binding of the receptor to the well was detected with enzyme-labelled antibodies.

Figure 5 shows Membrane Invasion Culture System (MICS) Assay of A375 cells in the presence of various concentrations of Mn²⁺ or Ca²⁺. A 30% increase in invasion is seen at 100µM MnCl₂.

Figure 6 shows inhibition of A375M invasion with GRGDSP in the presence of 100 µM CaCl₂ or 100 µM MnCl₂, using the MICS Assay.

The invention relates to the discovery that the presence of Mn²⁺ increases the binding avidity of integrins for their ligands. Such integrins include fibronectin, laminin, vitronectin and collagen receptors. It is now known that other cations as well modify the binding of integrins to their ligands. All integrin α subunits for which the amino acid sequence is known contain sequences that resemble known Ca²⁺-binding sites in other proteins. While not wishing to be bound by this explanation, it appears that the presence of these sequences renders the receptors sensitive to the presence of Mn²⁺.

Grinnell, Journal of Cell Science, V. 65 (1984), reports a study comparing Mn-dependent cell adhesion to non-specific substrates with cell adhesion to substrates coated with adhesive ligands. The author comes to the conclusion that integrins are not involved in Mn-dependent adhesion.

Integrins in general and their subunits are described in detail in Ruoslahti and Pierschbacher, Science 238:491 (1987). All terminology used herein conforms to the definitions and descriptions provided by this reference. In essence, these integrins comprise a family of related cell surface heterodimeric glycoproteins that are involved in mediating cell adhesive interactions. The integrins include, but are not limited to, receptors for fibronectin, vitronectin, collagens, laminin, tenascin, and the cell surface protein IIb/IIIa that recognizes fibrinogen, fibronectin, Von Willebrand factor and thrombospondin. The leukocyte adhesion receptors LFA-1, Mac-1 and gp 150/95 are also members of the integrin family of receptors.

For the purpose of this invention, the term "cations" shall include those ions having a positive charge, preferably 2+ or 3+. These include metals and the rare earth metals such as the lanthanide series.

As used herein, modification of receptor binding encompasses both changes in binding avidity and changes in binding affinity. Increased binding affinity reflects an increase in the strength of binding between individual receptors and their ligands. Increased binding avidity may reflect either increased binding affinity or an increase in the total number of receptor molecules binding to ligands.

The present invention relates to the striking effect which the presence of Mn²⁺ produces on the binding of the fibronectin receptor to fibronectin. Both the binding avidity and the binding affinity are greatly increased in the presence of Mn²⁺. Moreover, Mn²⁺ has been demonstrated to increase the binding avidity of other integrins examined for at least one of their ligands, as measured by one or more of the assays described in the Examples below. These integrins include the fibronectin, vitronectin, collagen Types I and IV and laminin receptors.

Other cations also exhibit effects on the binding avidity of the fibronectin receptor and other integrins. Such metals include, but are not limited to, Co²⁺, Cd²⁺, Ni²⁺ and Gd³+. The former three effect an increase in binding avidity. Gd³⁺ produces a decrease in binding avidity, and can be used to mitigate the integrin activity in general. Such modification of binding avidity can be detected by several different assays performed in the presence or absence of various concentrations of cations. Among these are assays to detect the binding of integrin-containing liposomes to particular ligands, the use of labelled antibodies to determine binding between integrins and ligands, the use of labelled integrins to determine integrin-ligand binding and affinity chromatography to determine the binding of integrins to a ligand-conjugated matrix. Because each of these assays may reflect different aspects of binding avidity, such as binding affinity or an increase in the number of receptors available for binding, the various assays may not all show a similar effect. Cations which affect the binding activity of integrins are considered to have integrin-binding-modifying activity. Where binding avidity is demonstrated by one or more assays to be increased in the presence of a cation, the cation is considered to have integrin-binding-promoting activity. Where binding avidity is demonstrated by one or more assays to be decreased in the presence of a cation, the cation is considered to have integrin-binding-inhibiting activity.

The demonstration that integrin-binding-promoting cations modify binding avidity of integrins has a number of applications. In order to modify binding avidity, integrin-binding-promoting cations may be provided, either alone or in conjunction with other cations, such as the physiologically prevalent Ca²⁺ and Mg²⁺. For example, the purification of integrins, such as on a ligand-conjugated matrix column, can be facilitated. Such column purification is described in Pytela, et al., PNAS 82:5766-5770 (1985). Briefly, a ligand reactive with the integrin of interest is conjugated to an appropriate support matrix, such as Sepharose^{R}, although other matrix material such as Sephadex^{R} or agarose may be used. Such ligands include intact integrin ligands, as well as fragments thereof, such as the 110 kD fragment of fibronectin, and synthetic Arg-Gly-Asp-containing peptides. A solution containing the desired integrin is passed over the column, thereby permitting the integrin to bind to the matrix. A solution capable of detaching the bound integrin from the matrix is then passed through the column, thereby eluting any bound integrin. In the presence of appropriate integrin-binding-promoting cation in the binding solution, the amount of integrin bound to the column is increased, thereby resulting in a greater yield of purified integrin.

Moreover, the increased integrin binding avidity in the presence of integrin-binding-promoting cations facilitates assays to determine the presence of either integrins or their ligands. For example, various immunoassay formats are available for determining the presence of binding pairs, such as a receptor-ligand, using labelled antibodies reactive with either the ligand or receptor. Such methods are well known to those skilled in the art. See for example HANDBOOK OF EXPERIMENTAL IMMUNOLOGY (D.M. Weir, Ed.), Blackwell Scientific Publications (3rd Ed. 1978). Appropriate labels include enzymes, components of a fluorescent, luminescent or chemiluminescent system, and radionuclides. Alternatively, a similarly labelled receptor or a labelled ligand may be utilized to determine the presence of the ligand or receptor, respectively, in the presence of integrin-binding-promoting cations.

Integrin-binding-promoting cations also facilitate cell migration. Such a result is particularly unexpected considering that cell migration requires both cell attachment and cell detachment. That factors which aid cell attachment would also aid cell migration does not necessarily follow. In particular Mn²⁺ was shown to increase cell migration both in an invasion type system and in a wound healing model. Integrin-binding-promoting cations, preferably Mn²⁺, can be applied directly where cell migration is desired, such as, for example, a wound or burn, to promote healing. The cation can be administered in a number of ways, such as by application of or immersion in a solution, by injection or by providing the cation in a suitable dressing, such as a bandage or internal reservoir. Moreover, adminstration in conjunction with a prosthesis or transplant can facilitate migration and attachment of host cells. Slow release or prolonged release methods of administration may be used.

The following examples are intended to illustrate but not limit the invention. Examples involving Ca⁺⁺ and/or Mg⁺⁺ alone in physiological concentrations are comparative examples.

### EXAMPLE I

### Mn²⁺ INCREASES THE BINDING OF FIBRONECTIN RECEPTOR LIPOSOMES TO FIBRONECTIN

The effect of cations on the binding of the fibronectin receptor to its ligand was studied by incorporating the receptor into liposomes and measuring receptor liposome attachment to a fibronectin-coated surface in the presence of various cations.

Fibronectin receptors were purified from human placenta by affinity chromatography on a 110 kDa chymotryptic fragment of fibronectin, according to the methods of Pytela et al., Cell 40:191 (1985), and Pytela et al., Meth. Enzymol. 144:475-489 (1987), with the following modifications. The 110 kDa fragment corresponds to the 120 kDa fragment described in Pytela et al. (1985), supra. One change from the original procedure was the use of Tris-buffered saline (TBS, 150 mM NaCl, 50 mM Tris-HCl, pH 7.3), instead of a phosphate buffer, to ensure solubility of the divalent cations. Briefly, homogenized placental tissue was washed once with 2 volumes of cold TBS containing 1 mM CaCl₂, 1 mM MgCl₂ and 3 mM phenylmethyl sulfonyl fluoride (PMSF), centrifuged, and the pellet was extracted with 1 volume of cold TBS containing 100 mM octyl-β-D-glucopyranoside (Calbiochem, La Jolla, CA), and calcium, magnesium, and PMSF as in Pytela et al., (1985) supra. The clear extract was slowly applied at 4°C, first to a column of plain Sepharose, and then to 110 kDa fibronectin fragment-Sepharose. The affinity column was washed at room temperature with 6 volumes of TBS containing 25 mM octyl-β-D-thioglucopyranoside (Calbiochem), and 1 mM CaCl₂ and 1 mM MgCl₂. Bound receptor was eluted with the wash buffer containing 20 mM EDTA in place of calcium and magnesium. Eluted receptor was used immediately for the preparation of receptor liposomes or stored frozen. In some experiments, 1 mM MnCl₂ was substituted for CaCl₂ and MgCl₂ throughout the isolation procedure.

Phosphatidylcholine liposomes were prepared and their attachment to surfaces assayed essentially as described in Pytela, et al. (1985), supra. Briefly, liposomes containing purified receptors were prepared by dialysis against TBS without cations. Aliquots of the resulting liposome preparation were mixed with the appropriate cations at the start of the assay in which liposomes were tested for attachment to plastic microtiter wells coated with various protein ligands. The proteins were coated onto the plastic at 25 µg/ml, a concentration known to give maximal liposome attachment. Specific attachment was calculated by subtracting the attachment to bovine serum albumin from the attachment to fibronectin and other ligands. The peptides tested for inhibition in the attachment assay were purified by high performance liquid chromatography according to Pierschbacher and Ruoslahti, J. Biol. Chem. 262:17294 (1987).

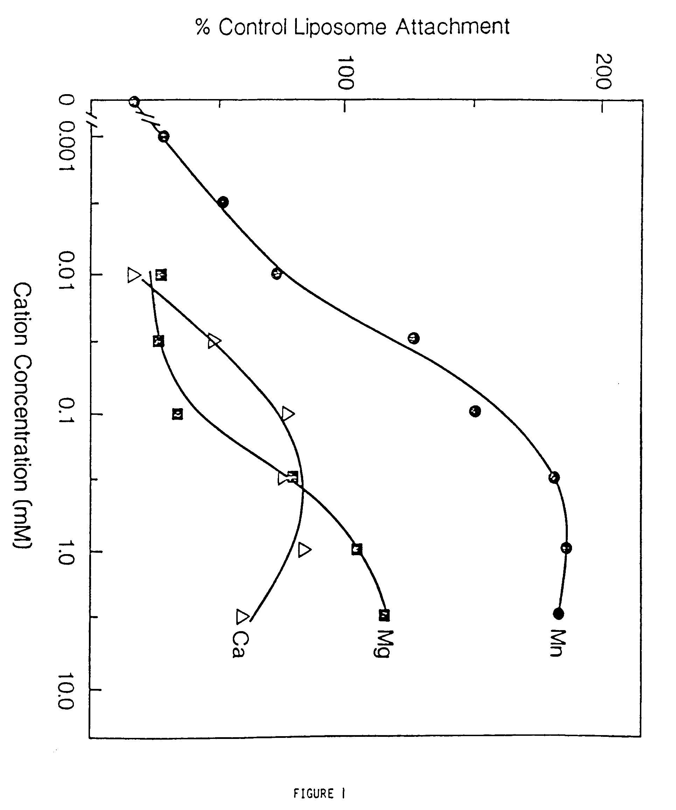

Cation titrations were performed to compare the effects of Mn²⁺ with those of Ca²⁺ and Mg²⁺, the most relevant physiological cations. As shown in Figure 1, these experiments demonstrated that Mn²⁺ increases receptor binding more effectively than either Mg²⁺ or Ca²⁺ over a wide range of concentrations, and that Mn²⁺ is effective at a much lower concentration than the other two cations. Mn²⁺ promoted liposome attachment at concentrations as low as 3 µM, near the concentration of Mn²⁺ in tissues. When liposome attachment was measured in the presence of 1 mM Ca²⁺ or Mg²⁺ and varying concentrations of Mn²⁺, the enhancement produced by Mn²⁺ was clearly demonstrable even when the Mn²⁺ concentration was only one-tenth of the Ca²⁺ or Mg²⁺ concentration (See Table I).

**TABLE I**

| EFFECT OF Mn²⁺ ON BINDING OF FIBRONECTIN RECEPTOR LIPOSOMES TO FIBRONECTIN IN THE PRESENCE OF Ca²⁺ OR Mg²⁺ | | |
|---|---|---|
| Mn²⁺ Added | 1mM Ca²⁺ | 1mM Mg²⁺ |
| None | 1.00* (comparison) | 1.00* (comparison) |
| 1 mM | 5.78 ± 0.47 | 3.12 ± 0.10 |
| 0.1mM | 4.41 ± 0.33 | 2.04 ± 0.08 |
| 0.01 mM | 1.23 ± 0.66 | 0.97 ± 0.17 |

| | | |
|---|---|---|
| *Results are expressed as a multiple of binding measured in Ca²⁺ or Mg²⁺ alone. Mean and standard error from two experiments is shown. | | |

### EXAMPLE II

### EFFECTS OF OTHER DIVALENT CATIONS

As can be seen in Table II, cations other than Mn²⁺ also affect the binding activity of fibronectin receptor liposomes to fibronectin, as prepared and assayed by the method of Example I. Specifically, Co²⁺ and Cd²⁺ increased the percentage of attachment above that obtained in the presence of Ca²⁺ and Mg²⁺. In one similar assay, Zn²⁺ produced attachment that was 128% of the control value. Ni²⁺ was as effective as the combination of Ca²⁺ and Mg²⁺. Four cations, Ba²⁺, Be²⁺, Fe²⁺ and Fe³⁺ produced less attachment than Ca²⁺, but did support fibronectin receptor attachment above the level found in the absence of either Ca²⁺ or Mg²⁺. The effect of Cu²⁺, Gd³⁺, La³⁺ and Sr²⁺ on fibronectin receptor attachment was difficult to determine, apparently in part because of solubility problems, but each one produced increased attachment above that found in the absence of Ca²⁺ or Mg²⁺ in at least one experiment. It was not possible to obtain a definitive result from Pb²⁺, again perhaps because of solubility problems.

**Table II**

| EFFECT OF CATIONS ON BINDING OF FIBRONECTIN RECEPTOR LIPOSOMES TO FIBRONECTIN | |
|---|---|
| Cation | % Attachment |
| Ca²⁺ and Mg²⁺ (comparison) | 100 |
| Ca²⁺ (comparison) | 75 ± 10 |
| Ni²⁺ | 100 ± 12 |
| Mg²⁺ | 110 ± 5 |
| Cd²⁺ | 130 ± 30 |
| Co²⁺ | 150 ± 13 |
| Mn²⁺ | 210 ± 29 |
| None | 9 ± 9 |

Aliquots of the receptor liposome preparation were mixed with the indicated cations (1 mM) and the attachment of the liposomes to microtiter wells coated with fibronectin was assayed. The results are expressed as a percentage of control attachment, which was measured in the presence of 1 mM CaCl₂ and 1 mM MgCl₂. Mean and standard error from three experiments are shown.

### EXAMPLE III

### Mn²⁺ CHANGES THE BINDING AFFINITY AND SPECIFICITY OF FIBRONECTIN RECEPTOR

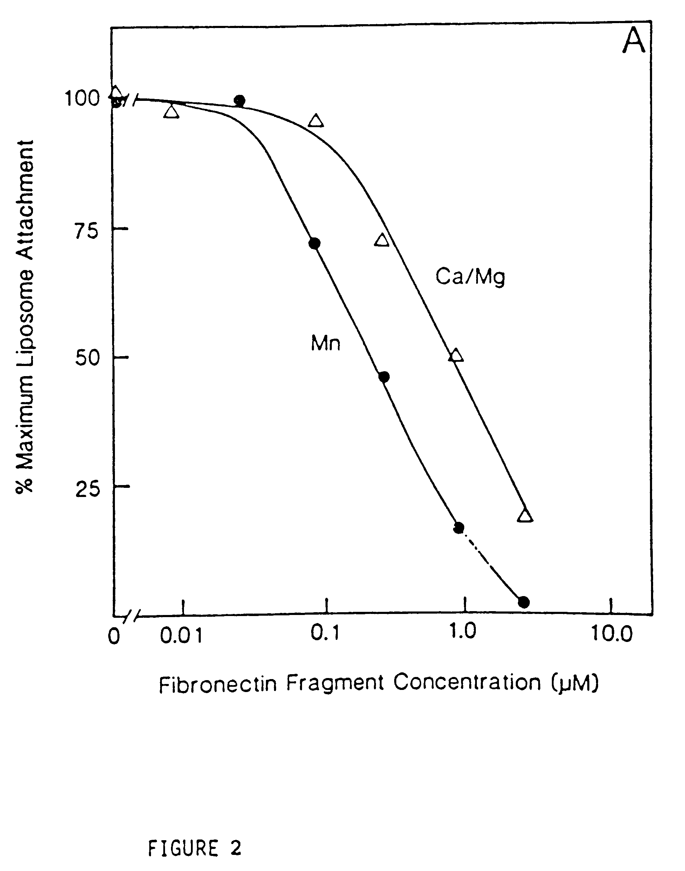

Inhibition of the receptor liposome attachment by soluble ligands was used to assay changes in the relative affinity of the receptor. The 110 kDa cell attachment fragment of fibronectin inhibited attachment of the receptor liposomes to fibronectin in a concentration dependent manner, as shown in Figure 2. It reduced binding by 50% at 2 X 10⁻⁷ M in the presence of Mn²⁺ and at 9 X 10⁻⁷ in the presence of Ca²⁺/Mg²⁺. A comparison of those concentrations indicates that Mn²⁺ increased the relative affinity of the receptor for the fragment by a factor of 4.5.

An Arg-Gly-Asp containing peptide, Gly-Arg-Gly-Asp-Ser-Pro (GRGDSP), also inhibited the attachment of the receptor liposomes to fibronectin. GRGDSP inhibited the binding of Mn²⁺-treated fibronectin receptor liposomes at much lower concentrations than those needed to inhibit the Ca²⁺/Mg²⁺-treated liposomes. The peptide concentration that gave 50% inhibition was 1.3 X 10⁻⁵ M in Mn²⁺ and 6.7 X 10⁻⁴ M in Ca²⁺/Mg²⁺. A comparison of the peptide concentrations that gave 50% inhibition indicates that Mn²⁺ increased the relative receptor affinity for the peptide by a factor of 50. These results demonstrate that Mn²⁺ increases the inhibitibility of the receptors for binding to the ligands. The inhibitory activity of the short synthetic peptides in particular is increased in the presence of Mn²⁺.

### EXAMPLE IV

### FIBRONECTIN RECEPTOR ELISA

An enzyme-linked immunosorbent assay, utilizing Mn²⁺, was developed to measure receptor binding activity.

Wells of plastic microtiter plates (Linbro, Titertek, Flow Laboratories) were coated with fibronectin or other proteins at 1µg/ml in Tris-buffered saline (TBS) overnight at room temperature. Before the binding assays, the plates were washed twice with TBS containing 0.01% Tween 20 (polyoxyethylene sorbitan monolaureate) (TBS-Tween) and twice with water. Fibronectin receptor was diluted in TBS containing 20mM octyl-β-D-thioglucopyranoside and 1mM MnCl₂. Receptor dilutions were incubated in fibronectin-coated wells for 90 minutes at room temperature. Non-bound receptor was washed away three times with TBS containing octylglucoside and Mn²⁺.

Bound receptor was detected by an incubation with purified rabbit anti-fibronectin receptor antibodies for 120 minutes at room temperature, followed by an overnight incubation with goat anti-rabbit IgG coupled to alkaline phosphatase (Sigma). Non-bound antibodies were removed by washing twice with TBS-Tween® and twice with water. Enzyme activity in the wells after the addition of the alkaline phosphatase substrate (1mg/ml of p-nitrophenylphosphate (Sigma), in 1M diethanolamine, pH 9.8, containing 1mM MgCl₂) was measured by multichannel photometer (Titertek, Multiscan, Flow Laboratories). Duplicate determinations were used for each assay. Controls included BSA-coated and non-coated wells.

In inhibition experiments, dilutions of fibronectin, fibronectin fragments, or peptides were incubated in fibronectin-coated microtiter wells in the presence of a constant concentration of fibronectin receptor (giving about 50% of the maximal binding in the direct binding assay). Bound receptor was detected as described above. When the effects of Ca²⁺ and Mg²⁺ were studied the assay was carried out using phosphate-buffered saline (PBS) instead of TBS, although it can be done in TBS as well.

Dose-dependent binding of fibronectin receptor to fibronectin was found in the ELISA. In the presence of Mn²⁺, the smallest amount of fibronectin receptor that could be detected was about 10 ng. Only minimal binding of fibronectin receptor to laminin, and no binding to vitronectin, bovine serum albumin or non-coated microtiter wells could be detected even at the highest concentration of the receptor. Negative controls showed that omission of the receptor, first antibody, or both, eliminated the signal on fibronectin. Titration of the fibronectin used for the coating showed that the optimal coating concentration of fibronectin was 1µg/ml. This coating concentration together with an amount of receptor that gave about 50% of the maximal binding was used in the subsequent inhibition experiments.

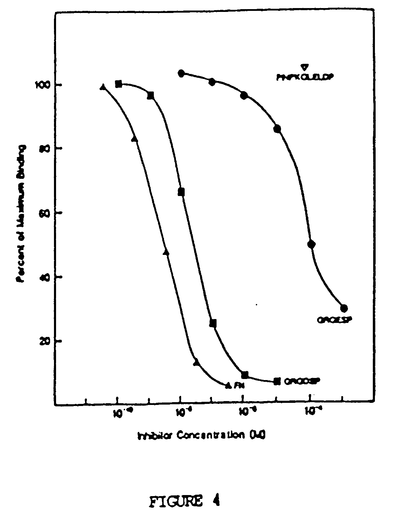

Fibronectin, the 110 kDa and 11.5 kDa (Pytela et al. (1987)) cell attachment fragments, and the GRGDSP peptide derived from the fibronectin cell attachment site were tested for their capacity to inhibit the binding of fibronectin receptor to solid-phase fibronectin in ELISA. The 110 kDa fragment of fibronectin had the highest inhibitory activity; half-maximal inhibition (ID₅₀) was obtained at approximately 5 X 10⁻⁹ M. The inhibitory capacities of the other ligands decreased in the order of fibronectin > GRGDSP > 11.5-kDa fragment and the ID₅₀ were 8.6 X 10⁻⁹ M, 8.8 X 10⁻⁸ M and 7.7 X 10⁻⁷ M, respectively. See Figure 4 which shows inhibition by fibronectin and the peptide GRGDSP. It also shows that the closely-related peptide GRGESP is 2000 times less inhibitory than GRGDSP and that an unrelated peptide is essentially devoid of activity.

The assays described above were carried out in the presence of Mn²⁺ to enhance the binding of the receptor to fibronectin. In the presence of the physiological divalent cations, Ca²⁺ and Mg²⁺, the assay was less sensitive. This observation confirmed the increased affinity of the receptor for its ligand in the presence of Mn²⁺ relative to Ca²⁺ and Mg²⁺.

An alternative method of detection used radio-labelled receptors in place of the antibodies. The assays were carried out in removable microtiter wells (Immunolon 2, Removawell, Dynatech Laboratories). The coating efficiency was studied by incubating concentrations of 0.1, 0.5, 1, 5 and 10µg/ml of ¹²⁵I-fibronectin overnight in wells. Nonbound fibronectin was washed away as in the ELISA and the radioactivity remaining in the wells was counted.

To find the optimal coating concentration of fibronectin for receptor binding, the same concentrations of unlabeled fibronectin as above were used to coat the wells, followed by the addition of a constant concentration of ¹²⁵I-labeled fibronectin receptor (250ng/ml). After a 90 minute incubation at room temperature, non-bound receptor was washed away as in the ELISA procedure, and the bound radioactivity was counted. There was a dose dependent binding of ¹²⁵I-labeled fibronectin receptor to the wells.

The affinity constant between fibronectin and fibronectin receptor was determined by a displacement experiment. A single concentration of ¹²⁵I-fibronectin receptor (250ng/ml) was used and its displacement was measured as a function of increasing concentrations of the nonradioactive receptor (0-25µg/ml). The coating concentration of fibronectin was 1µg/ml and non-specific binding was defined as the amount of radioactive receptor, which bound to wells coated with 1µg/ml of BSA. The apparent dissociation constant was 3 X 10⁻⁸ M.

### EXAMPLE V

### Mn²⁺ INCREASES THE YIELD OF FIBRONECTIN RECEPTOR FROM AFFINITY CHROMATOGRAPHY

Fibronectin receptor can be isolated by affinity chromatography on the 110 kDa fibronectin fragment (See Pytela, et al., (1985), (1987), supra). However, the yields are low because the receptor binds to the affinity matrix loosely and is partially removed by the washing buffer. The yield of the receptor from the affinity chromatography was improved by substituting 1 mM Mn²⁺ for Ca²⁺/Mg²⁺ in the chromatography buffers. The yield, estimated by densitometry of bands from SDS-PAGE analysis, was four-fold higher with Mn²⁺ than with Ca²⁺ and Mg²⁺, as shown in Table III. A Gelman densitometer was used to scan gels stained with Coomassie blue. Three similar experiments showed that this increase in yield was representative. Also, Mn²⁺ produced a comparable yield when 0.5% Nonidet P-40 (Calbiochem) was used as a detergent in place of octyl-β-D-glucopyranoside Receptor preparations isolated with Mn²⁺ or Ca²⁺/Mg²⁺ were similar to one another in SDS-PAGE. When the receptor isolated with Mn²⁺ was tested in the liposome attachment assay, the binding of the receptor was several times higher in Mn²⁺ than in Ca²⁺/Mg²⁺, suggesting that the effect of Mn²⁺ on the receptor is reversible.

**TABLE III**

| DENSITOMETRY OF SDS-PAGE ANALYSIS OF FIBRONECTIN RECEPTOR ISOLATED IN THE PRESENCE OF Ca²⁺/Mg²⁺ OR Mn²⁺ | |
|---|---|
| Cations | Receptor Peak Area |
| 1 mM Ca²⁺ and 1 mM Mg²⁺ (comparison) | 4.7 |
| 1 mM Mn²⁺ | 18.2 |

### EXAMPLE VI

### Mn²⁺ INCREASES THE BINDING OF OTHER INTEGRINS TO LIGANDS

Vitronectin receptor was purified from human placenta as described in Example I for the fibronectin receptor, but a different affinity matrix containing the peptide Gly-Arg-Gly-Asp-Ser-Pro-Lys was employed. The peptide was synthesized with an Applied Biosystems Model 430A, according to the manufacturer's instructions. Vitronectin receptor was isolated according to the method of Example I in the presence of 1 mM Ca²⁺ and 1mM Mg²⁺ or in 1 mM Ca²⁺ alone. In a number of liposome attachment assays, Mn²⁺ enhanced the binding of this integrin to vitronectin by 20-50% over the binding measured in Ca²⁺ and Mg²⁺.

Type I collagen receptor was also isolated from placenta, employing the method given in Example I with the following modifications. Mn²⁺ was used in the chromatography buffers for this procedure, and the affinity matrix contained Type I collagen. Type I collagen receptor liposomes treated with Ca²⁺ and Mg²⁺ did not measurably bind to collagen. However, clear and specific binding to collagen was measured in the presence of Mn²⁺.

### EXAMPLE VII

### MODULATION OF CELL MIGRATION IN CELL INVASION ASSAYS

The effect of Mn²⁺ on cell migration was determined by examining tumor cells as an indicative system. The invasiveness was determined using a modification of the Membrane Invasion Culture System (MICS) as described in Hendrix et al. (1985) Clin. Exp. Metastasis 3:221-223, which is incorporated herein by reference. The cell line used was a human melanoma cell line designated A375M, the derivation of which has been described in Kozlowski et al., J.N.C.I. 72:913 (1984), which is incorporated herein by reference. The cells were selected for the tests because they are known to migrate through the membrane system in easily quantifiable numbers. Noninvasive fibroblasts were used as control cells. All of the cells were cultured in Dulbecco's Modified Eagle's Medium (DMEM) (Gibco, Chagrin Falls, OH) supplemented with 10% heat inactivated fetal bovine serum (Tissue Culture Biologicals, Tulare, CA) and 0.1% gentamicin (Gibco). Cells were removed from culture dishes using 2 mM EDTA in PBS devoid of Ca²⁺ and Mg²⁺ for all assays. Cells were radiolabelled with 0.25 µCi/ml ¹⁴C-thymidine (New England Nuclear, Boston, MA) for 48 hours in DMEM containing 2% fetal bovine serum and then seeded, either in the presence of absence of MnCl₂ into the upper compartment of the MICS chambers prepared as follows.

Fresh human placentae were obtained at birth, and in each case the amnion was obtained from near the region of the umbilical cord. After several rinses in sterile PBS, Fungi-zone/penicillin/streptomycin (Irvine Scientific, Irvine, CA) and PBS again, the amnion was trimmed to fit specially designed MICS chambers as depicted in Gehlsen and Hendrix, (1987) Pigment Cell 1:16-21, Figure 1, which is incorporated herein by reference. The amnion was aseptically interposed between the top and bottom plates of the MICS apparatus, with the epithelial surface facing the top plate. The fastening screw was tightened and the extra membranous material trimmed away with a scalpel. Before fitting the membrane in the chamber, the bottom wells (the "target wells") were filled with sterile DMEM (Gibco) containing 10% fetal bovine serum. The amniotic epithelium was removed by treatment with freshly prepared 0.25 M ammonium hydroxide (NH₄OH) for 5 minutes at room temperature followed by extensive washing in PBS, which left a denuded basement membrane with an underlying collagenous stroma. In this manner, the interaction of cells with an extracellular matrix could be studied without the interference of host cells.

The labelled cells at a final concentration of 1.0 X 10⁵/ml were delivered into each upper, or "seeding," chamber in serum-containing DMEM and placed in a humidified incubator at 37°C with 5% CO₂ and 95% air atmosphere. All membranes were carefully examined for leakiness prior to cell seeding.

The number of cells able to successfully invade the basement membrane and underlying collagenous stroma was determined by removing the media in the lower wells (1.1 ml) after each 24 hour increment and at 72 hours via the side ports in the MICS chambers without disruption of the ongoing experiment. After each lower well was sampled, fresh media was replenished in the lower wells. In this manner, cell invasion could be assessed repeatedly in the same experiment over various time intervals. The ¹⁴C-thymidine radio-labelled cells in all the lower well samples were pelleted and then placed in scintillation vials. In all experiments, the collected ¹⁴C-labelled cells were then lysed with 0.5 ml of 1 N NaOH. Aliquots of 100µl glacial acetic acid were added to each vial to prevent chemiluminescence, and 10 ml of ACS scintillation cocktail (Amersham Corporation, Arlington Heights, IL) was added prior to radiolabel determination using a scintillation counter (Beckman Instruments, Brea, CA).

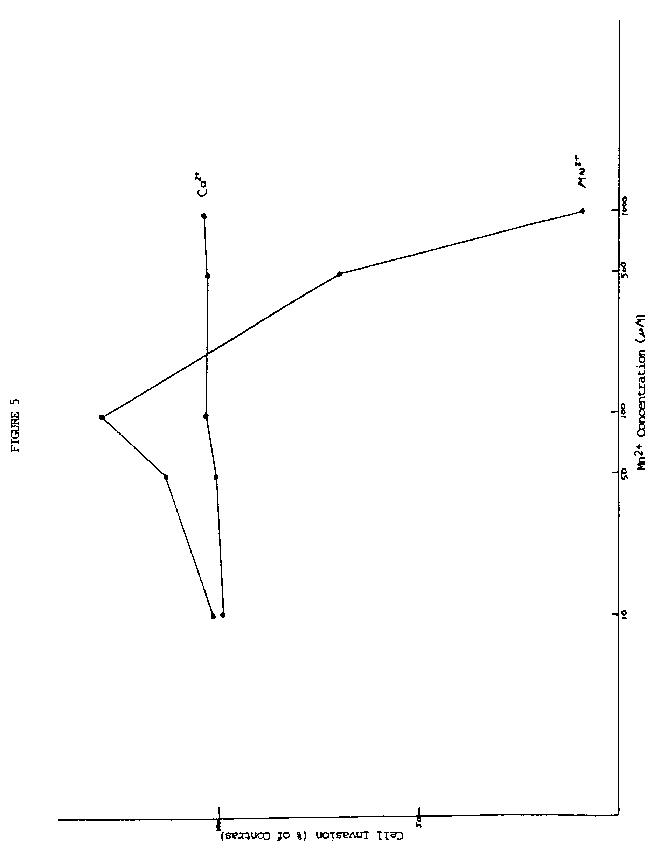

The modulation of amnion membrane migration by Mn²⁺ was tested by performing the assay as described above, with MnCl₂ at various concentrations (10 to 1000µM) included into both the upper and lower wells of the MICS chambers. The ability of the A375M cells to invade the amnion in either Mn²⁺ or Ca²⁺ is shown in Figure 5.

These results show that at a concentration of 100 µM, Mn²⁺ increased the migration of the A375M cells by 30% after 72 hours. At higher concentrations of Mn²⁺, cell viability is affected and therefore migration is reduced.

### EXAMPLE VIII

### ENHANCED INHIBITION OF TUMOR CELL INVASION BY ADHESION PEPTIDES IN THE PRESENCE OF Mn²⁺

Peptides containing the RGD sequence have been shown to inhibit the invasion of tumor cells through the amniotic membrane in the MICS system. This effect is thought to be due to the inhibition of the fibronectin receptor function by the peptides (Gehlsen et al., JCB 106:925-930 (1988)). Since Mn²⁺ increases the affinity of the fibronectin receptor for the GRGDSP more than for fibronectin, the effect of the synthetic peptide was tested in the invasion assay described above, in the presence of Mn²⁺. GRGDSP was added to the invasion assay in a titrated fashion (10-1000µg/ml) with either 100µM MnCl₂ or CaCl₂. Cell invasion was thus calculated after 72 hours.

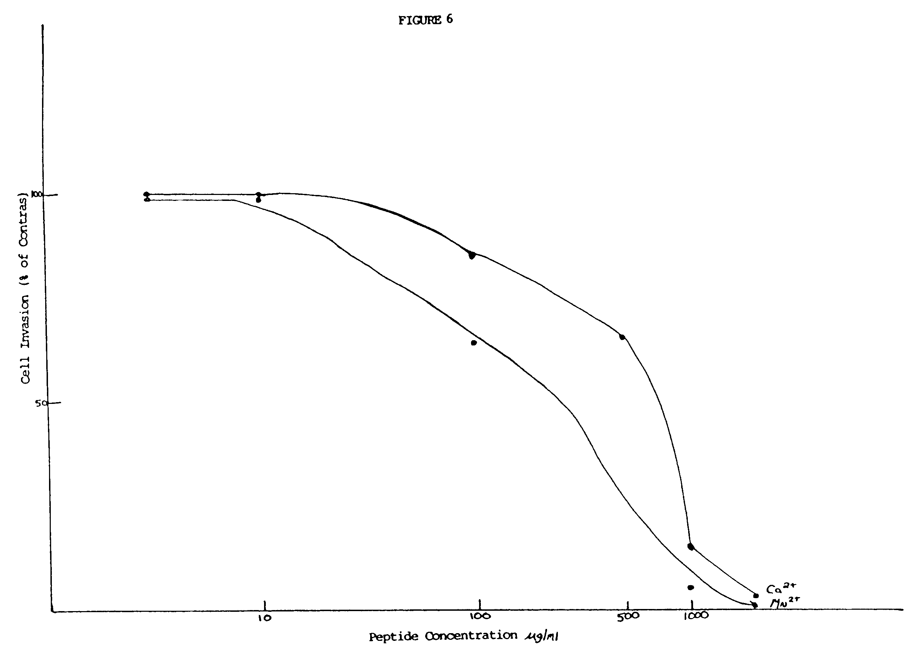

The results in Figure 6 show that the peptide concentration necessary to give 50% inhibition of invasion was 5 X 10⁻⁵ M in Mn²⁺ compared to 1.2 X 10⁻⁴ in Ca²⁺. A control peptide, GRGESP (E is Glutamic Acid), had no effect in either cation. The data show that less peptide is required to inhibit invasion in the presence of Mn²⁺, relative to Ca²⁺.

### EXAMPLE IX

### MODULATION OF CELL MIGRATION IN AN IN VITRO "WOUND" MODEL

An in vitro wounding assay was developed to test the effects of Mn²⁺ on cell migration into a wounded (cell free) area that was coated with various adhesion proteins. This assay allowed the determination of the specific ligands and the respective receptors that are involved in the migration process. More specifically, this assay was used to test cell migration on various substrates in the presence of Mn²⁺. A375M cells were grown to confluency in 6 well culture dishes (Corning, New York, NY). A specially designed comb which scratches out 4-2mm wide lanes was used to make a crossing pattern in the cell layer, removing the cells. The cell-free lanes were then coated for 1 hour at 37°C with one of the following ligands: fibronectin, vitronectin, Type I or Type IV collagen, bovine serum albumin or laminin. These proteins were dissolved in PBS at a concentration of 10µg/ml and then added directly to the wounded cell layer.

Standard Culture Media containing Ca²⁺ and Mg²⁺ and 100 µM MnCl₂ was added to the culture dishes while control dishes had the same media without Mn²⁺. Cell migration was monitored by visualization.

In the presence of Mn²⁺, A375M cells exhibited augmented migration or filling in of the free spaces coated with fibronectin and Type IV collagen over that of controls. These data show that the migration of cells on substrates can be augmented by providing Mn²⁺.

### EXAMPLE X

### Gd³⁺ DECREASES THE BINDING OF INTEGRINS TO THEIR LIGANDS

The ability of Gd³⁺ to inhibit the binding of integrins was assayed in the liposome attachment system. Fibronectin receptor (FNR) and vitronectin receptor (VNR) liposomes were prepared by dialysis against TBS containing 1 mM CaCl₂ and 1 mM MgCl₂. Aliquots of GdCl₃ were added to the liposomes at the start of the assay. At concentrations above 0.3 mM, Gd³⁺ caused a significant decrease in attachment of fibronectin receptor and vitronectin receptor to their ligands, as shown in Table IV. These results suggest that Gd³⁺ is a general inhibitor of the integrin function and that it can be used to mitigate undesirable effects of integrin activity. Such situations include binding of inflammatory cells at the site of a tissue injury caused by, for example, an ischemic episode, such as is associated with a cardiac or brain infarct.

**TABLE IV**

| INHIBITION BY Gd³⁺ OF INTEGRIN BINDING | | |
|---|---|---|
| Gd³⁺ Concentration (mM) | % Inhibition | |
| | FNR | VNR |
| 3 | 87 | 100 |
| 1 | 91 | 93 |
| 0.3 | 61 | 59 |
| 0.1 | 8 | 0 |
| 0 | 0 | 0 |

## Claims

1. Use of cations having integrin-binding-modifying activity for the manufacture of a medicament for promoting wound healing wherein the binding avidity or affinity of an integrin for its ligand has been modified by providing an effective amount of a divalent or trivalent cation having integrin-binding-modifying activity with the proviso that said divalent or trivalent cation is not Zn²⁺ or Al³⁺, nor a physiological concentration of Ca²⁺ or Mg²⁺.

2. The use of claim 1, wherein said cation is selected from Mn²⁺, Co²⁺, Cd²⁺, Ni²⁺, Zn²⁺ and Gd³⁺.

3. The use of claim 1, wherein said modifying comprises increasing the binding avidity of said integrins for their ligands.

4. The use of claim 1, wherein said modifying comprises decreasing the binding avidity of said integrins for their ligands.

5. The use of claim 1, wherein said cation is Mn²⁺.

6. The use of claim 4, wherein said cations are of the lanthanide series.

7. The use of claim 1, wherein said modifying comprises increasing the binding of an integrin from solution to a ligand-coated surface by providing Mn²⁺.

8. The use of claim 1, wherein said modifying comprises increasing the binding of a ligand to an integrin receptor in solution by providing an integrin-binding-promoting cation.

9. The use of claim 8, wherein said ligand is a peptide containing the amino acid sequence Arg-Gly-Asp wherein said peptide has cell-attachment-promoting activity.

10. The use of claim 8, wherein said cation is a divalent cation.

11. The use of claim 8, wherein said cation is selected from Mn²⁺, Co²⁺, Ni²⁺ and Zn²⁺.

12. The use of claim 1, wherein said integrin is in solution.

13. The use of claim 12, wherein said cation is a divalent or trivalent cation.

14. The use of claim 12, wherein said cation is selected from Mn²⁺, Co²⁺, Cd²⁺, Ni²⁺, Zn²⁺ and Gd³⁺.

15. The use of claim 12, wherein said modifying comprises increasing the binding avidity of said integrin for its ligand.

16. The use of claim 1, wherein said integrin is in a liposome vesicle.

17. The use of claim 16, wherein said cation is selected from Mn²⁺, Co²⁺, Cd²⁺, Ni²⁺, Zn²⁺ and Gd³⁺.

18. The use of claim 16, wherein said modifying comprises increasing the binding avidity of said integrin for its ligand.

19. The use of claim 1, wherein said modifying comprises decreasing the binding affinity of said integrins for their ligands.

20. The use of claim 1, wherein said integrins are selected from fibronectin, vitronectin, laminin, type I collagen and type IV collagen receptors.

21. The use of claim 1, wherein Mn²⁺ is selected for increasing the binding avidity of the fibronectin, vitronectin, collagen type I and/or collagen type II receptors for their respective ligands.

22. In a method of purifying receptors by passing them through and eluting them from a column having a matrix to which is attached an integrin ligand, the improvement comprising providing an integrin-binding-promotion cation in the binding solution.

23. The method of claim 22, wherein the integrin-binding-promoting cation further comprises Mn²⁺.

24. The method of claims 22 or 23, wherein said ligand is the 110kD fibronectin fragment.

25. The method of claims 22 or 23, wherein said ligand is a peptide containing the amino acid sequence Arg-Gly-Asp, wherein said peptide has cell attachment promoting activity.

26. The method of claims 22 to 25, wherein said improvement further comprises eluting said receptors with EDTA.

27. The use of claim 8, wherein said ligands are selected from fibronectin, vitronectin, type I collagen and type IV collagen.

28. A method of detecting the binding of integrins to their ligands, comprising the steps of:
a) permitting said integrins to bind to said ligands in the presence of integrin-binding-modifying cations;
b) detecting said integrin-ligand binding.

29. The method of claim 28 wherein said integrin-ligand binding is detected by providing labelled antibodies reactive with said integrin-ligand complexes.

30. A method for detecting the binding of integrins to their ligands, comprising the steps of:
a) labelling said integrins so as to be detectable; and
b) permitting said labelled integrins to bind to said ligands in the presence of integrin-binding-promoting cations;
c) detecting the presence of said bound labelled integrins.

31. The method of claim 30 wherein said detectable label is selected from the group consisting of radionuclides, enzymes, fluorescent or luminescent molecules.

32. A method of determining the presence of an integrin, comprising the steps of:
a) permitting said integrin to bind to an immobilized ligand in the presence of an integrin-binding-promoting cation;
b) providing antibodies reactive with said integrin, wherein said antibodies are capable of detection; and
c) determining binding between said antibodies and said integrin.

33. A method of determining the presence of an integrin ligand, comprising the steps of;
a) permitting an integrin to bind to said integrin ligand; and
b) determining binding between said integrand and said ligand.

34. A method of promoting cell migration by providing integrin-binding-promoting cations.

35. The method of claim 34 wherein said integrin-binding-promoting cations are divalent cations.

36. The method of claim 34 wherein said integrin-binding-promoting cations are selected from Mn²⁺, Co²⁺, Cd²⁺, and Ni²⁺.

37. A method of inhibiting binding of an integrin to a ligand by providing Mn²⁺ and a soluble ligand, said ligand including the amino acid sequence Arg-Gly-Asp and having cell attachment promoting activity.

## Patentansprüche

1. Verwendung von Kationen mit einer die Integrinbindung modifizierenden Aktivität zur Herstellung eines Medikaments zur Förderung der Wundheilung, bei der die Bindungsavidität oder -affinität eines Integrins für seinen Liganden modifiziert worden ist durch Bereitstellung einer wirksamen Menge eines bivalenten oder trivalenten Kations mit einer die Integrinbindung modifizierenden Aktivität unter der Voraussetzung, daß das bivalente oder trivalente Kation nicht Zn²⁺ oder Al³⁺ oder eine physiologische Konzentration von Ca²⁺ oder Mg²⁺ ist.

2. Verwendung nach Anspruch 1, bei der das Kation ausgewählt wird aus Mn²⁺, Co²⁺, Cd²⁺, Ni²⁺, Zn²⁺ und Gd³⁺.

3. Verwendung nach Anspruch 1, bei der die Modifikation eine Steigerung der Bindungsavidität des Integrins für seine Liganden umfaßt.

4. Verwendung nach Anspruch 1, bei der die Modifikation die Verringerung der Bindungsavidität des Integrins für seine Liganden umfaßt.

5. Verwendung nach Anspruch 1, bei der das Kation Mn²⁺ ist.

6. Verwendung nach Anspruch 4, bei der die Kationen der Lanthanoidengruppe angehören.

7. Verwendung nach Anspruch 1, bei der die Modifikation die Steigerung der Bindung eines Integrins aus einer Lösung an eine mit Liganden beschichtete Oberfläche durch Bereitstellung von Mn²⁺ umfaßt.

8. Verwendung nach Anspruch 1, bei der die Modifikation eine Steigerung der Bindung eines Liganden an einen Integrin-Rezeptor in Lösung durch Bereitstellung eines die Integrinbindung fördernden Kations umfaßt.

9. Verwendung nach Anspruch 8, bei der der Ligand ein Peptid ist, das die Aminosäuresequenz Arg-Gly-Asp enthält, wobei das Peptid eine die Zellanheftung fördernde Aktivität besitzt.

10. Verwendung nach Anspruch 8, bei der das Kation ein bivalentes Kation ist.

11. Verwendung nach Anspruch 8, bei der das Kation ausgewählt wird aus Mn²⁺, Co²⁺, Ni²⁺ und Zn²⁺.

12. Verwendung nach Anspruch 1, bei der das Integrin in Lösung vorliegt.

13. Verwendung nach Anspruch 12, bei der das Kation ein bivalentes oder trivalentes Kation ist.

14. Verwendung nach Anspruch 12, bei der das Kation ausgewählt wird aus Mn²⁺, Co²⁺, Cd²⁺, Ni²⁺, Zn²⁺ und Gd³⁺.

15. Verwendung nach Anspruch 12, bei der die Modifikation eine Steigerung der Bindungsavidität des Integrins für seinen Liganden umfaßt.

16. Verwendung nach Anspruch 1, bei der das Integrin ein Liposomenvesikel ist.

17. Verwendung nach Anspruch 16, bei der das Kation ausgewählt wird aus Mn²⁺, Co²⁺, Cd²⁺, Ni²⁺, Zn²⁺ und Gd³⁺.

18. Verwendung nach Anspruch 16, bei der die Modifikation eine Steigerung der Bindungsavidität des Integrins für seinen Liganden umfaßt.

19. Verwendung nach Anspruch 1, bei der die Modifikation eine Verringerung der Bindungsaffinität des Integrins für seine Liganden umfaßt.

20. Verwendung nach Anspruch 1, bei der die Integrine ausgewählt werden aus Fibronektin-, Vitronektin-, Laminin-, Typ I-Kollagen- und Typ VI-Kollagen-Rezeptoren.

21. Verwendung nach Anspruch 1, bei der Mn²⁺ ausgewählt wird zur Steigerung der Bindungsavidität der Fibronektin-, Vitronektin-, Kollagen Typ I- und/oder Kollagen Typ II-Rezeptoren für ihre jeweiligen Liganden.

22. Verfahren zur Reinigung von Rezeptoren durch Passage derselben durch und Elution derselben von einer Säule mit einer Matrix, an der ein Integrinligand haftet, umfassend die Bereitstellung eines die Integrinbindung fördernden Kations in der Bindungslösung.

23. Verfahren nach Anspruch 22, bei dem das die Integrinbindung fördernde Kation ferner Mn²⁺ umfaßt.

24. Verfahren nach Anspruch 22 oder 23, bei dem der Ligand das Fibronektinfragment von 110 kD ist.

25. Verfahren nach Anspruch 22 oder 23, bei dem der Ligand ein Peptid ist, das die Aminosäuresequenz Arg-Gly-Asp enthält, wobei das Peptid eine die Zellanheftung fördernde Aktivität besitzt.

26. Verfahren nach den Ansprüchen 22 bis 25, das ferner das Eluieren der Rezeptoren mit EDTA umfaßt.

27. Verwendung nach Anspruch 8, bei der die Liganden ausgewählt werden aus Fibronektin, Vitronektin, Typ I-Kollagen und Typ IV-Kollagen.

28. Verfahren zum Nachweis der Bindung von Integrinen an ihre Liganden, bei dem man:
a) den Integrinen die Bindung an die Liganden in Gegenwart von die Integrinbindung modifizierenden Kationen gewährt,
b) die Integrin-Liganden-Bindung nachweist.

29. Verfahren nach Anspruch 28, bei dem die Integrin-Liganden-Bindung nachgewiesen wird, indem markierte Antikörper bereitgestellt werden, die gegenüber den Integrin-Liganden-Komplexen reaktiv sind.

30. Verfahren zum Nachweis der Bindung von Integrinen an ihre Liganden, bei dem man:
a) die Integrine markiert, um sie nachweisbar zu machen, und
b) den markierten Integrinen die Bindung an die Liganden in Gegenwart von die Integrinbindung fördernden Kationen gewährt,
c) die Anwesenheit des gebundenen markierten Integrins nachweist.

31. Verfahren nach Anspruch 30, bei dem der nachweisbare Marker ausgewählt wird aus der Gruppe bestehend aus Radionukliden, Enzymen, fluoreszierenden oder lumineszierenden Molekülen.

32. Verfahren zur Bestimmung der Anwesenheit eines Integrins, bei dem man:
a) dem Integrin die Bindung an einen immobilisierten Liganden in Anwesenheit eines die Integrinbindung fördernden Kations gewährt,
b) Antikörper bereitstellt, die gegenüber dem Integrin reaktiv sind, wobei die Antikörper zum Nachweis geeignet sind, und
c) die Bindung zwischen den Antikörpern und dem Integrin nachweist.

33. Verfahren zum Nachweis der Anwesenheit eine Integrinliganden, bei dem man:
a) einem Integrin die Bindung an den Integrinliganden gewährt, und
b) die Bindung zwischen dem Integrin und dem Liganden nachweist.

34. Verfahren zur Förderung der Zellmigration durch Bereitstellung von die Integrinbindung fördernden Kationen.

35. Verfahren nach Anspruch 34, bei dem die die Integrinbindung fördernden Kationen bivalente Kationen sind.

36. Verfahren nach Anspruch 34, bei dem die die Integrinbindung fördernden Kationen ausgewählt werden aus Mn²⁺, Co²⁺, Cd²⁺ und Ni²⁺.

37. Verfahren zur Hemmung der Bindung eines Integrins an einen Liganden durch Bereitstellung von Mn²⁺ und einem löslichen Liganden, wobei der Ligand die Aminosäuresequenz Arg-Gly-Asp einschließt und eine die Zellanheftung fördernde Aktivität besitzt.

## Revendications

1. Utilisation de cations possédant une activité modifiant la liaison à l'intégrine dans la préparation d'un médicament destiné à favoriser la cicatrisation dans lequel l'avidité ou l'affinité de liaison d'une intégrine pour son ligand a été modifiée par addition d'une quantité efficace d'un cation di- ou trivalent possédant une activité modifiant la liaison de l'intégrine sous réserve que ledit cation di- ou trivalent ne soit pas le Zn²⁺ ou l'Al³⁺ ni une concentration physiologique de Ca²⁺ ou de Mg²⁺.

2. Utilisation selon la revendication 1, dans laquelle ledit cation est choisi au sein du groupe constitué du Mn²⁺, du Co²⁺, du Cd²⁺, du Ni²⁺, Zn²⁺ et le Gd³⁺.

3. Utilisation selon la revendication 1, dans laquelle lesdites modifications comprennent une augmentation de l'avidité de liaison desdites intégrines pour leurs ligands.

4. Utilisation selon la revendication 1, dans laquelle lesdites modifications comprennent une diminution de l'avidité de liaison desdites intégrines pour leurs ligands.

5. Utilisation selon la revendication 1, dans laquelle ledit cation est le Mn²⁺.

6. Utilisation selon la revendication 4, dans laquelle lesdits cations appartiennent à la série des lanthanides.

7. Utilisation selon la revendication 1, dans laquelle ladite modification comprend une augmentation de la liaison d'une intégrine en solution à une surface recouverte d'un ligand par addition de Mn²⁺.

8. Utilisation selon la revendication 1, dans laquelle ladite modification comprend une augmentation de la liaison d'un ligand à une intégrine en solution par addition d'un cation favorisant la liaison de l'intégrine.

9. Utilisation selon la revendication 8, dans laquelle ledit ligand est un peptide contenant la séquence d'acides aminés Arg-Gly-Asp dans laquelle ledit peptide a une activité favorisant l'attachement des cellules.

10. Utilisation selon la revendication 8, dans laquelle ledit cation est un cation divalent.

11. Utilisation selon la revendication 8, dans laquelle ledit cation est choisi parmi le Mn²⁺, le Co²⁺, le Ni²⁺ et le Zn²⁺.

12. Utilisation selon la revendication 1, dans laquelle ladite intégrine est en solution.

13. Utilisation selon la revendication 12, dans laquelle ledit cation est un cation di- ou trivalent.

14. Utilisation selon la revendication 12, dans laquelle ledit cation est choisi parmi le Mn²⁺, le Co²⁺, le Cd^{2,} le Ni²⁺, le Zn²⁺ et le Gd³⁺.

15. Utilisation selon la revendication 12, dans laquelle ladite modification comprend une augmentation de l'avidité de liaison de ladite intégrine pour son ligand.

16. Utilisation selon la revendication 1, dans laquelle ladite intégrine est une vésicule liposomale.

17. Utilisation selon la revendication 16, dans laquelle ledit cation est choisi parmi le Mn²⁺, le Co²⁺, le Cd^{2,} le Ni²⁺, le Zn²⁺ et le Gd³⁺.

18. Utilisation selon la revendication 16, dans laquelle ladite modification comprend une augmentation de l'avidité de liaison de ladite intégrine pour son ligand.

19. Utilisation selon la revendication 1, dans laquelle ladite modification comprend une diminution de l'affinité de liaison desdites intégrines pour leurs ligands.

20. Utilisation selon la revendication 1, dans laquelle lesdites intégrines sont choisies parmi les récepteurs de la fibronectine, de la vitronectine, de la laminine et du collagène de type I et IV.

21. Utilisation selon la revendication 1, dans laquelle le Mn²⁺ est choisi pour augmenter l'avidité de liaison des récepteurs de la fibronectine, de la vitronectine, du collagène de type I et/ou du collagène de type IV pour leurs ligands respectifs.

22. Procédé de purification des récepteurs consistant à les faire passer sur une colonne, dont ils sont ensuite élués, la colonne contenant une matrice à laquelle est attachée un ligand de l'intégrine, l'amélioration comprenant l'addition à la solution de liaison d'un cation favorisant la liaison de l'intégrine.

23. Procédé selon la revendication 22, dans lequel le cation favorisant la liaison de l'intégrine comprend en outre le Mn²⁺.

24. Procédé selon la revendication 22 ou 23, dans lequel ledit ligand est le fragment de 110 kDa de la fibronectine.

25. Procédé selon la revendication 22 ou 23, dans lequel ledit ligand est un peptide contenant la séquence d'acides aminés Arg-Gly-Asp, où ledit peptide a une activité favorisant l'attachement des cellules.

26. Procédé selon les revendications 22 à 25, dans lequel ladite amélioration comprend en outre l'élution desdits récepteurs avec l'EDTA

27. Utilisation selon la revendication 8 dans laquelle lesdits ligands sont choisis parmi la fibronectine, la vitronectine, le collagène de type I et le collagène de type IV.

28. Procédé de détection de la liaison des intégrines à leurs ligands comprenant les étapes consistant à :
a) permettre auxdites intégrines de se lier auxdits ligands en présence de cations modifiant la liaison des intégrines; et
b) détecter ladite liaison intégrine-ligand.

29. Procédé selon la revendication 28 dans lequel ladite liaison intégrine-ligand est détectée en ajoutant des anticorps marqués réagissant avec lesdits complexes intégrine-ligand.

30. Procédé de détection de la liaison des intégrines à leurs ligands comprenant les étapes consistant à :
a) marquer lesdites intégrines de manière à ce qu'elles soient détectables;
b) permettre auxdites intégrines de se lier auxdits ligands en présence de cations favorisant la liaison des intégrines; et
c) détecter la présence desdites intégrines marquées liées.

31. Procédé selon la revendication 30 dans lequel ledit marqueur détectable est choisi au sein du groupe comprenant des radionuclides, des enzymes et des molécules fluorescentes ou luminescentes.

32. Procédé de détermination de la présence d'une intégrine comprenant les étapes consistant à :
a) permettre à ladite intégrine de se lier à un ligand immobilisé en présence d'un cation favorisant la liaison des intégrines;
b) ajouter des anticorps réagissant avec ladite intégrine où lesdits anticorps sont capables d'être décelés; et
c) déterminer la liaison desdits anticorps et de ladite intégrine.

33. Procédé de détection de la présence d'un ligand d'une intégrine comprenant les étapes consistant à :
a) permettre à l'intégrine de se lier audit ligand de l'intégrine; et
b) déterminer la liaison entre ladite intégrine et ledit ligand.

34. Procédé pour faciliter la migration cellulaire en ajoutant des cations facilitant la liaison ligand-intégrine.

35. Procédé selon la revendication 34 dans lequel lesdits cations favorisant la liaison ligand-intégrine sont des cations divalents.

36. Procédé selon la revendication 34 dans lequel les cations favorisant la liaison ligand-intégrine sont des cations choisis parmi le Mn²⁺, le Co²⁺, le Cd²⁺ et le Ni²⁺.

37. Procédé d'inhibition de la liaison d'une intégrine à un ligand en ajoutant du Mn²⁺ et un ligand soluble, ledit ligand comprenant la séquence d'acides aminés Arg-Gly-Asp et possédant une activité favorisant l'attachement des cellules.
